(19) Europäisches Patentamt
European Patent Office
Office européen des brevets

(11) Publication number: **0 064 646 B1**

(12) # EUROPEAN PATENT SPECIFICATION

(45) Date of publication of patent specification: **27.02.91**

(51) Int. Cl.[5]: **A61K 31/135**

(21) Application number: **82103450.1**

(22) Date of filing: **23.04.82**

Divisional application 89115176.3 filed on 23/04/82.

(54) **Use for the manufacture of oral forms of nadolol for treating glaucoma.**

(30) Priority: **24.04.81 US 257453**
**14.05.81 US 263656**
**25.06.81 US 277378**

(43) Date of publication of application:
**17.11.82 Bulletin 82/46**

(45) Publication of the grant of the patent:
**27.02.91 Bulletin 91/09**

(84) Designated Contracting States:
**AT BE CH DE FR GB IT LI LU NL SE**

(56) References cited:
**EP-A- 0 037 622**
**FR-A- 2 330 388**
**US-A- 3 982 021**

**CHEMICAL ABSTRACTS, vol. 81, no. 21, 25th November 1974, page 35, no. 131068y, Columbus Ohio (USA);**

**Br. Med. J., 1978, 2, p. 1089 / ref. cited in "Martindale" (1982), p. 1333 - see "Migraine"**

(73) Proprietor: **E.R. Squibb & Sons, Inc.**
**Lawrenceville-Princeton Road**
**Princeton, N.J. 08540-4000(US)**

(72) Inventor: **Sudilovsky, Abraham**
**712 Winchester Ave**
**Lawrenceville, N.J.(US)**
Inventor: **Muir, John Gordon**
**R.R. 1, Box 342 Poor Farm Road**
**Pennington, N.J.(US)**

(74) Representative: **Vossius & Partner**
**Siebertstrasse 4 P.O. Box 86 07 67**
**D-8000 München 86(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to the use of nadolol or an ester thereof or a pharmaceutically acceptable acid-addition salt for the preparation of drugs for oral administration for treating glaucoma or lowering intraocular pressure.

The term "nadolol" as employed herein refers to the beta blocker 2,3-cis-1,2,3,4-tetrahydro-5-[2-hydroxy-3-(tert-butylamino)propoxy]-2,3-naphthalenediol (and pharmaceutically acceptable acid-addition salts thereof)

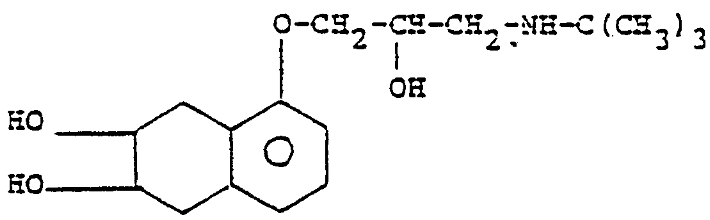

which is disclosed in U.S. Patent Nos. 3,935,267 and 3,982,021.

Esters or nadolol included herein are the mono-, di- and tri-esters (and pharmaceutically acceptable acid-addition salts thereof).

The di-esters have the structure

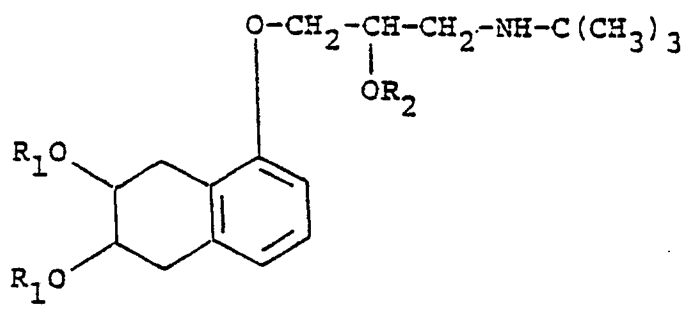

wherein both $R_1$ groups are acyl, preferably acetyl, and $R_2$ is hydrogen.

Esters or nadolol wherein both $R_1$ groups are hydrogen and $R_2$ is acyl (mono-ester), preferably acetyl, and wherein both $R_1$ groups are acyl, preferably acetyl, and $R_2$ is acyl, preferably acetyl (tri-esters) are disclosed in British Patent Specification GB-A-1,559,987.

In carrying out the method of the present invention, the nadolol or nadolol ester or a physiologically acceptable acid-addition salt may be administered to mammalian species, such as monkeys, dogs, cats and rats and as such may be incorporated in a conventional dosage form such as a tablet, capsule or elixir, along with the necessary carrier material, excipient, lubricant or buffer. Single or divided doses of about 8 to about 120 mg, preferably about 20 to 80 mg/one to four times daily may be administered in dosage forms as described above.

Example 1

A nadolol formulation suitable for oral administration in the treatment of glaucoma is set out below.

| Ingredient | mg/tablet |
|---|---|
| Nadolol | 40 |
| Magnesium stearate | 1 |
| Microcrystalline cellulose | 72 |

The nadolol is blended with the microcrystalline cellulose in a Hobart-type mixer for 5 minutes. Thereafter, the magnesium stearate is added with mixing for 2-3 minutes. The final mix is compressed in a

Strokes D3 tablet press to form a 40 mg tablet which is used for treating glaucoma or reducing intraocular pressure.

## Claims

1. The use of nadolol or an ester thereof or a pharmaceutically acceptable acid-addition salt for the preparation of drugs for oral administration for treating glaucoma or lowering intraocular pressure.

2. The use as defined in claim 1 wherein said ester is a mono-, di- or tri-ester.

3. The use as defined in claim 2 wherein said ester is a monoacetate, diacetate or triacetate.

4. The use as defined in claim 1 wherein said nadolol or ester thereof is administered in a dosage of from about 8 to about 120 mg.

5. The use as defined in claim 4 wherein said nadolol or ester thereof is administered in a dosage of from about 20 to about 80 mg.

6. The use as defined in claim 1 wherein said nadolol or ester thereof is in the form of a pharmaceutically acceptable acid-addition salt thereof.

## Revendications

1. Utilisation du nadolol ou d'un ester du nadolol ou d'un sel d'addition d'acide pharmaceutiquement acceptable pour la préparation de médicaments à administration orale servant à traiter le glaucome ou à abaisser la pression intra-oculaire.

2. Utilisation telle que définie dans la revendication 1, dans laquelle ledit ester est un mono-, un di- ou un tri-ester.

3. Utilisation telle que définie dans la revendication 2, dans laquelle ledit ester est un monoacétate, un diacétate ou un triacétate.

4. Utilisation telle que définie dans la revendication 1, dans laquelle ledit nadolol ou ester de nadolol est administré à une dose d'environ 8 à environ 120 mg.

5. Utilisation telle que définie dans la revendication 4, dans laquelle ledit nadolol ou ester de nadolol est administré à une dose d'environ 20 à environ 80 mg.

6. Utilisation telle que définie dans la revendication 1, dans laquelle ledit nadolol ou ester de nadolol se présente sous la forme d'un sel d'addition d'acide pharmaceutiquement acceptable.

## Ansprüche

1. Verwendung von Nadolol oder einem Ester davon oder einem pharmazeutisch verträglichen Säureadditionssalz zur Herstellung eines oral verabreichbaren Arzneimittels zur Behandlung von Glaukom oder zur Verminderung des Augeninnendrucks.

2. Verwendung nach Anspruch 1, wobei der Ester ein Mono-, Di- oder Triester ist.

3. Verwendung nach Anspruch 2, wobei der Ester ein Monoacetat, Diacetat- oder Triacetatester ist.

4. Die Verwendung nach Anspruch 1, wobei das Nadolol oder der Ester davon in einer Dosis von etwa 8 bis etwa 120 mg verabreicht wird.

5. Verwendung nach Anspruch 4, wobei das Nadolol oder der Ester davon in einer Dosis von etwa 20 bis 80 mg verabreicht wird.

6. Die Verwendung nach Anspruch 1, wobei das Nadolol oder der Ester davon in Form eines pharmazeutisch verträglichen Säureadditionssalzes vorliegt.